# EUROPEAN PATENT APPLICATION

(11) **EP 4 497 820 A1**
(43) Date of publication of application: **29.01.2025**
(21) Application number: 23774882.7
(22) Date of filing: 20.03.2023
(51) Int. Cl.: C12N 5/073, C12M 1/00, C12Q 1/02

(54) **EMBRYO CULTURE METHOD**

(30) Priority: 23.03.2022 JP 2022047454
(71) Applicant: Kyushu University, National University Corporation, Nishi-ku Fukuoka-shi Fukuoka 819-0395 (JP)
(72) Inventor: MENO Chikara, Fukuoka-shi, Fukuoka 819-0395 (JP); NII Takenobu, Fukuoka-shi, Fukuoka 819-0395 (JP)
(74) Representative: Hoffmann Eitle
(86) International application number: PCT/JP2023/010943
(87) International publication number: WO 2023/182287

(57) **Abstract**

The present invention provides a method for culturing an embryo. More specifically, the present invention comprises culturing an embryo (particularly pre-implantation) on a dish having a sufficiently thick extracellular matrix layer on the surface thereof. The present invention makes it possible to develop an embryo into, for example, a post-implantation embryo.

## Description

### Technical Field

The present invention relates to a method for culturing an embryo.

### Background Art

Methods for culturing an embryo *in vitro* have been developed for investigating embryonic development and infertility. Non Patent Literature 1 discloses a method for culturing a mouse blastocyst up to the stage of the egg-cylinder embryo stage. Non Patent Literature 1, however, does not disclose that the cultured embryo gastrulates. Non Patent Literature 1 discloses that the embryonic development more efficiently proceeds up to the egg-cylinder embryo stage by resecting a part of an embryo (E4.5) but an intact embryo cannot be normally developed into the later stage.

Non Patent Literature 2 discloses a method for culturing a post-implantation embryo, and more specifically, discloses that an embryo (E5.5 or E6.5) was successfully developed into E11.

### Citation List

### Non Patent Literatures

Non Patent Literature 1: Bedzhov et al., Nature Protocols 9, 2732-2739, 2014
Non Patent Literature 2: Aguilera-Castrejon et al., Nature 593, 119-124, 2021

### Summary of Invention

The present invention provides a method for culturing an embryo.

The method disclosed in Non Patent Literature 1 corresponds to a method for culturing a pre-implantation embryo up to the egg-cylinder embryo stage, whereas the method of Non Patent Literature 2 corresponds to a method for culturing a post-implantation embryo. However, a method for reproducing the embryonic development from pre-implantation to the post-implantation (for example, gastrulation process) has not yet been known. Then, the present invention provides a condition and method for culturing an embryo from the pre-implantation development phase to the gastrulation process *in vitro.* The present invention provides a method for developing an embryo without destruction of the embryo.

According to the present invention, the following inventions are provided.
(1) A method for culturing an embryo *in vitro,* comprising
   providing a culture dish having an inner bottom surface coated with an extracellular matrix layer on which the embryo is placed, wherein the embryo is contained in a sufficient amount of a medium suitable for culturing the embryo, and then
   culturing the embryo placed on the extracellular matrix layer under a condition suitable for culturing the embryo, wherein
   a thickness of the extracellular matrix layer from the inner bottom surface to a top surface of the extracellular matrix layer in contact with the embryo is equal to or more than a predetermined thickness;
   the predetermined thickness is sufficient to prevent adhesion of the embryo onto the inner bottom surface during culture or adhesion of the embryo and expansion of an adhesion area thereof on the inner bottom surface;
   the embryo is an embryo in a first development phase between a blastocyst stage and a gastrula stage,
   thereby successfully developing the embryo in the blastocyst stage up to a second development phase between the blastocyst stage and the gastrula stage; and the second development phase is the same development phase as the first development phase or a later development phase.
(2) The method according to the above (1), wherein the medium is a serum-free medium.
(3) The method according to the above (1) or (2), wherein the medium is a chemically defined medium.
(4) The method according to any of the above (1) to (3), wherein a site on which the embryo is to be placed is within a hole formed in a top surface of the extracellular matrix layer.
(5) The method according to the above (4), wherein the embryo is placed so as to be in contact with an inner wall of the hole.
(6) The method according to any of the above (1) to (5), wherein the embryo is placed on the extracellular matrix layer such that a mural trophectoderm thereof faces up or down.
(7) The method according to any of the above (1) to (6), wherein the first development phase is a late blastocyst and the second development phase is a gastrula.
(8) The method according to any of the above (1) to (7), wherein
   the medium contains a test compound,
   the method further comprises
   confirming whether or not growth of the embryo cultured in the presence of the test compound differs from growth of the embryo cultured in the absence of the test compound,
   if there is a difference, the test compound is determined to have an effect on development of the embryo in the first development phase to the second development phase, and/or
   if there is no difference, the test compound is determined to have no effect on development of the embryo in the first development phase to the second development phase.
(9) The method according to any of the above (1) to (7), further comprising
   culturing a test embryo in the first development phase under a condition where a normal embryo in the first development phase develops from the first development phase to the second development phase, wherein
   if the test embryo is not normally developed from the first development phase to the second development phase, the test embryo is determined to have an abnormality in development of the embryo in the first development phase to the second development phase, and/or
   if the test embryo is normally developed from the first development phase to the second development phase, the test embryo is determined to have an ability to develop from the first development phase to the second development phase.
(10) A culture system for use in culturing an embryo, comprising
   a culture dish for culturing the embryo, the culture dish having an inner bottom surface coated with an extracellular matrix layer, wherein
   a thickness of the extracellular matrix layer from the inner bottom surface to a top surface of the extracellular matrix layer in contact with the embryo is equal to or more than a predetermined thickness;
   the predetermined thickness is sufficient to prevent adhesion of the embryo onto the bottom surface during culture or adhesion of the embryo and expansion of an adhesion area thereof on the bottom surface;
   the embryo is an embryo in a first development phase between a blastocyst stage and a gastrula stage,
   thereby successfully developing the embryo in the blastocyst stage up to a second development phase between the blastocyst stage and the gastrula stage; and the second development phase is the same development phase as the first development phase or a later development phase.

### Brief Description of Drawings

[Figure 1A] Figure 1A shows a petri dish having a multiwell plate placed therein.
[Figure 1B] Figure 1B shows a multiwell plate and a sectional view of a well provided in the multiwell plate.
[Figure 1C] Figure 1C shows a petri dish having a multiwell plate placed therein.
[Figure 1D] Figure 1D shows the sectional view of a well having no coating on the inner bottom surface thereof.
[Figure 1E] Figure 1E shows the sectional view of a well having an extracellular matrix membrane on the inner bottom surface thereof.
[Figure 1F] Figure 1F shows a sectional view of a well having a thick extracellular matrix membrane on the inner bottom surface thereof, for use in 3D on top culture.
[Figure 1G] Figure 1G shows a sectional view of a well having an extracellular matrix having recesses on the inner bottom surface, for use in 3D in hole culture.
[Figure 2A] Figure 2A shows an optical microscope image of an embryo (an embryo (E4.5)) of the late blastocyst stage cultured for a day in wells having the inner bottom surface with no coating and the immunohistochemical staining results thereof. In Figure 2A, it is observed that the embryo adheres to the inner bottom surface and the adhesion areas expand outward from the embryo. Also in Figure 2A, it is observed that the trophectoderm extends along the culture surface outward from the embryo.
[Figure 2B] Figure 2B shows an optical microscope image of an embryo in the late blastocyst stage cultured for a day in a well having extracellular matrix membrane on the inner bottom surface and the immunohistochemical staining results thereof. In Figure 2B, it is observed that the embryo adheres to the inner bottom surface having extracellular matrix membrane and the adhesion area expands outward from the embryo. Also in Figure 2B, it is observed that the trophectoderm extends along the culture surface outward from the embryo.
[Figure 2C] Figure 2C shows an optical microscope image of an embryo in the late blastocyst stage cultured by 3D on top culture for a day and the immunohistochemical staining results thereof. In Figure 2C, it is observed that the embryo keeps its shape and does not adhere to the inner bottom surface.
[Figure 2D] Figure 2D shows an optical microscope image of an embryo in the late blastocyst stage cultured by 3D in hole culture for a day and the immunohistochemical staining results thereof. In Figure 2D, it is observed that the embryo keeps its shape and does not adhere to the inner bottom surface.
[Figure 3] Figure 3 shows the immunohistochemical staining results of embryos of the late blastocyst stage cultured by 3D in hole culture for two days.
[Figure 4A] Figure 4A shows the immunohistochemical staining results of embryos of the late blastocyst stage cultured by 3D in hole culture for three days.
[Figure 4B] Figure 4B shows the immunohistochemical staining results of an embryo obtained by culturing embryos of the late blastocyst stage in IVCM containing human serum by 3D in hole culture for three days.
[Figure 4C] Figure 4C shows the immunohistochemical staining results of an embryo obtained by culturing the embryo in the late blastocyst stage in IVCM containing human serum by 3D in hole culture for four days.
[Figure 5] Figure 5 shows the immunohistochemical staining results of an embryo obtained by culturing a blastocyst (E3.5 embryo) for a day and culturing for 3 days by 3D on top culture.
[Figure 6A] Figure 6A shows a sectional view of a culture dish having an extracellular matrix layer for use in 3D on top culture.
[Figure 6B] Figure 6B shows a sectional view of a culture dish having an extracellular matrix layer for use in 3D in hole culture.
[Figure 6C] Figure 6C shows the structure of a blastocyst.
[Figure 7] Figure 7 shows the configuration of a culture system.
[Figure 8] Figure 8 shows the results of 3D in hole culture of an embryo (E4.5) in a well containing a hydrogel modified with a laminin-derived peptide for 72 hours. The culture was carried out in a xeno-free environment.
[Figure 9A] Figure 9A shows the results of 3D in hole culture of an embryo (E4.5) in a well containing Gelrite containing a basement-membrane extracellular matrix (BME) for 24 hours.
[Figure 9B] Figure 9B shows the results of 3D in hole culture of an embryo (E4.5) in a well containing Gelrite containing recombinant human vitronectin (VTN-N) for 24 hours. The culture on VTN-N coat-containing Gelrite was performed in a xeno-free environment.
[Figure 10A] Figure 10A shows embryos obtained by culturing a primate (common marmoset) blastocyst for 3 days, hatching it from the zona pellucida, followed by culturing for 5 days in hIVCM containing 4-hydroxyestradiol and further in hIVCM for 15 days. Culture was performed for 20 days.
[Figure 10B] Figure 10B shows images of immunostained embryos obtained by culturing for 20 days.

### Detail Description of the invention

In the specification, "embryo" may be an embryo of a mammal including a primate such as a human, a gorilla, a chimpanzee, an orangutan, a bonobo, a marmoset, a rhesus monkey and a cynomolgus monkey; a rodent such as a mouse, a rat, and a hamster; a carnivore such as a dog and a cat; a perissodactyl such as a horse, a donkey and a mule; and an artiodactyl such as a cow, a pig, a llama, an alpaca, a goat and sheep. In an embodiment, the embryo is not an embryo of a marsupial.

In the specification, "in *vitro"* refers to outside the body, and particularly means performing culture in a culture solution artificially prepared. In contrast, "in *vivo"* refers to inside the body and "in utero" refers to inside the uterus.

In the specification, "extracellular matrix" refers to a substance present in the outside of a cell, filling the extracellular space and playing a role as a physical support for the cell. The extracellular matrix is usually insoluble. Examples of the extracellular matrix of a vertebrate such as a human include collagen, proteoglycan, fibronectin, vitronectin and laminin. The extracellular matrix for the connective tissue (interstitial tissue) is rich in type I collagen, proteoglycan (e.g., versican, decorin) and fibronectin. Examples of the basement-membrane extracellular matrix include type IV collagen, heparan sulfate proteoglycan, laminin, and entactin. In an embodiment, the basement-membrane extracellular matrix contains laminin. The basement membrane is present between the epithelium and the connective tissue.

In the specification, "culture" refers to maintaining a cell, a cell aggregate, and/or an embryo under the conditions suitable for culture, growing, or allowing them to differentiate into a later development phase. The culture is usually performed in a medium suitable for culture. The medium contains nutrients required for culture and can maintain a cell, a cell aggregate, and/or an embryo, grow, or allow them to differentiate into a later development phase. Since the medium loses nutrients and waste materials accumulate in the medium with the progress of culture, the medium is exchanged with a fresh one after a predetermined time. Examples of the medium include a serum-containing medium, a serum-free medium and a chemically defined medium. The serum-free medium is a medium containing no serum. The chemically defined medium is a medium containing components defined in type and amount. The serum-free medium and chemically defined medium can be albumin-free mediums. The albumin-free medium does not contain albumin. Examples of other mediums include a xeno-free medium and a cytokine-free medium. The xeno-free medium refers to a medium containing no heterologous components, particularly components derived from a heterologous animal.

According to the present invention, there is provided a method for culturing an embryo *in vitro.* According to the present invention, there are also provided a method for growing an embryo *in vitro* and a method for allowing an embryo to differentiate into a later development phase *in vitro.* An embryo develops from the blastocyst stage to the gastrula stage via the late blastocyst stage and the egg-cylinder embryo stage. In an embodiment, the embryo can be an embryo in the late blastocyst stage. In an embodiment, the embryo is a naturally occurring embryo. In an embodiment, the embryo is artificially produced. In an embodiment, the embryo can be a blastoid (embryo-like structure). In an embodiment, the embryo can be an ETX embryo. The blastoid can be an embryo obtained by co-culturing, for example, an ES cell, trophoblast stem cells (TS cell) and extraembryonic endoderm cells (XEN cells)(embryo in the development phase corresponding to the late blastocyst stage, an embryo in the development phase corresponding to the egg-cylinder embryo stage, or an embryo in the development phase corresponding to the gastrula stage (see, for example, Sozen B., et al., Nature Cell Biology, 20: 979-989, 2018). It is known that the embryo proposed by Sozen, 2018 is not a complete embryo. According to the method of the present invention, a pre-implantation embryo (for example, embryo in the late blastocyst stage) and an embryo in the later development phase thereof can be grown or developed into a post-implantation embryo (for example, an embryo in the gastrula stage) *in vitro.*

The method of the present invention may include culturing an embryo *in vitro.* The method of the present invention may preferably include developing an embryo into a later development stage *in vitro.* The method of the present invention may include growing or developing a pre-implantation embryo (for example, an embryo in the late blastocyst stage) and an embryo in a later development phase thereof into a post-implantation embryo (for example, an embryo in the gastrula stage) *in vitro.*

An embryo in the blastocyst stage develops into an embryo in the gastrula stage via the late blastocyst stage and the egg-cylinder embryo stage. In the method of the present invention, the embryo may be an embryo in a first development phase between the blastocyst stage (particularly late blastocyst) and the gastrula stage. In a preferred embodiment, the embryo in a first development phase may be an embryo in the blastocyst stage (more specifically, blastocyst, preferably late blastocyst). The blastocyst corresponds to an embryo in the blastula stage having a cleavage cavity, which results from repeated cleavage of the fertilized egg. The blastocyst has a globular appearance and contains a blastocoel (i.e., cleavage cavity) and inner cell mass, which are covered with trophectoderm. The blastocyst is implanted when it is surrounded with the endometrial epithelium, *in vivo.* The gastrula is an embryo formed when epiblasts derived from the inner cell mass invaginate at the primitive streak. The gastrula having three cell layers, ectoderm, mesoderm, and endoderm, is formed by gastrulation. That is, if the ectoderm, mesoderm or endoderm is present in an embryo, it is found that the embryo is at least in the development phase after the gastrula. In a preferred embodiment, the first development phase is a pre-implantation stage and a second development phase is a post-implantation stage.

In the method of the present invention, an embryo can be grown up to a second development phase between the blastocyst stage (particularly late blastocyst stage) and the gastrula stage. The second development phase herein is the same development phase as the first development phase or a later development phase. The second development phase is preferably the later phase than the first development phase and can be a day or more, 2 days or more, 3 days or more, or 4 days or more later development phase. For example, in a preferred embodiment, the embryo in the second development phase is a gastrula.

Up to the present, there have been no research reports that an embryo in the blastocyst stage is developed into a gastrula having the primitive streak by culturing the embryo *in vitro.* The present invention revealed that an embryo in the blastocyst stage can be developed into a gastrula by culturing the embryo in a predetermined condition. In the method of the present invention, an embryo can be cultured while it is kept intact *in vitro* (more specifically, kept unbroken). The method of the present invention does not include damaging or destroying an embryo even partially or completely. In the present invention, the parietal endoderm and the ventral endoderm are formed from the primitive endoderm of an embryo cultured and Reichert's membrane can be formed from the parietal endoderm.

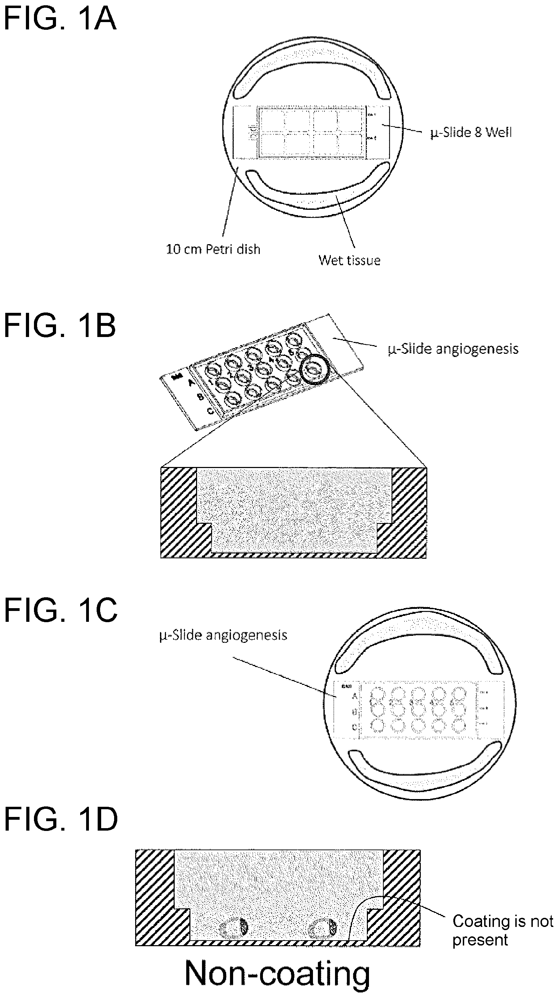

In the method of the present invention, an embryo can be cultured in a culture dish having an inner bottom surface coated with an extracellular matrix layer. The culture dish has a container-like shape that can store a liquid medium for culturing an embryo. The culture dish generally has a bottom, a side wall extending from the outer periphery of the bottom to the top and an opening on the top, and can store a liquid such as a culture solution (see, for example, Figure 1B). An embryo is cultured in a culture solution stored in the culture dish. The size of the culture dish is not particularly limited as long as it is available for culturing an embryo. A culture dish having an opening of 3 mm to 10 mm, for example, 3 mm to 5 mm, can be preferably used. The opening may be covered with a lid (covering the opening) during culture. In an embodiment, the culture dish may be a well provided in a multiwell plate. The multiwell plate has a plurality of wells arranged in a single plate-like substrate.

When a blastocyst is cultured on the inner bottom surface having no extracellular matrix layer, the blastocyst adheres onto the inner bottom surface during culture and the adhesion area expands in the periphery. If the extracellular matrix layer is present but thin, similarly the blastocyst adheres onto the inner bottom surface during culture and the adhesion area expands in the periphery. If so, the embryo cannot develop into a gastrula. Although not bound by theory, when embryonic cells adhere onto the inner bottom surface, the embryo is destroyed, with the result that normal development of the embryo is likely to be prevented. The bottom of the dish may be directly coated with the extracellular matrix layer but a support layer may be present between the extracellular matrix layer and the bottom of the dish. The support layer is not particularly limited as long as it (for example, solid support) can support the extracellular matrix layer stably and has no unacceptable cytotoxicity. For example, a biocompatible support may be acceptable. A support layer may contain a support selected from the group consisting of agar and hydrogel such as gellan gum. The extracellular matrix can be laminated on the support layer or immobilized onto the surface of the support layer. When the support has a hole (deep and opened) for introducing an embryo, the inner surface of the hole is desirably coated with the extracellular matrix layer, and at least a part in contact with an embryo is the extracellular matrix layer. The embryo can be in contact with the bottom and/or side surface of the hole during culture. In the case where the hole has an approximately cylindrical shape, an embryo can be in contact with the bottom and side surfaces. In the case where the hole has an approximately conical shape, an embryo can be in contact with the side surface. In the case where the hold has an approximately spherical shape, an embryo can be in contact with one or more points or regions of the curved surface. The opening or inner peripheral portion is not particularly limited but it may be, for example, about 50 µm to 200 µm, for example, about 70 µm to 150 µm. The extracellular matrix layer may contain a biocompatible diluent in addition to an extracellular matrix. Examples of the biocompatible diluent include an aqueous solution, a buffer solution, a physiological saline, and a medium. The extracellular matrix layer may be constituted of a mixture (gel-like composition) of an effective amount of an extracellular matrix and a support (preferably, non-adhesive to a cell, for example, hydrogel). On the extracellular matrix layer containing an effective amount of an extracellular matrix, an embryo can be normally developed (for example, up to the gastrula stage, preferably the late gastrula stage).

In the method of the present invention, the blastocyst is cultured in a culture dish having an inner bottom surface coated with a sufficiently thick extracellular matrix layer. More specifically, in the present invention, a thickness of the extracellular matrix layer from the inner bottom surface to a top surface of the extracellular matrix layer in contact with an embryo is equal to or more than a predetermined thickness. The predetermined thickness is the thickness sufficient or necessary to prevent adhesion of the embryo onto the bottom surface during culture or adhesion of the embryo and expansion of an adhesion area thereof on the bottom surface. The thickness is sufficient to allow three-dimensional culture (3D culture). In three-dimensional culture, a scaffold may or may not be used. In the present invention, an extracellular matrix is used as a scaffold for culturing an embryo. Examples of the extracellular matrix include a hydrogel extracted from an animal extracellular matrix, a protein hydrogel, a peptide hydrogel and a polymer hydrogel. As the extracellular matrix, a hydrogel extracted from the basement membrane can be used. In a preferred embodiment, the extracellular matrix is one or more, more preferably, 2 or more, further preferably, 3 or more, and still further preferably all selected from the group consisting of type IV collagen, heparan sulfate proteoglycan, laminin, and entactin. In the present invention, preferably a hydrogel extracted from an animal extracellular matrix can be used as the extracellular matrix, and more preferably, a hydrogel extracted from the basement membrane can be used as the extracellular matrix. These extracellular matrixes can be xeno-free. The term "xeno-free" means that a heterologous component, particularly a component of a different animal species, is not contained. In a preferred embodiment, examples of the extracellular matrix include matrigel^{™} and Cultrex^{™} UltiMatrix Reduced Growth Factor, BME001. These can be preferably used in the culture disclosed in the present invention. Matrigel^{™} and UltiMatrix Reduced Growth Factor are a solubilized basement-membrane extracellular matrix secreted by Engelbreth-Holm-Swarm mouse sarcoma cells. In a preferred embodiment, the extracellular matrix may contain laminin, collagen IV, entactin, and heparan sulfate proteoglycan. In a preferred embodiment, the extracellular matrix may contain 35 to 40% (for example, about 39%) of laminin, 35 to 50% (for example, about 45%) of collagen IV and 10 to 150 (for example, about 130) of entactin, and further contain heparan sulfate proteoglycan. Since entactin strengthens a structure of a hydrogel of laminin and collagen IV, the extracellular matrix can acquire high tensile strength by addition of entactin. Introduction of entactin is advantageous for 3D in hole culture or 3D on Top culture. In the present invention, the extracellular matrix layer may contain an effective amount of an extracellular matrix or a component or partial peptide thereof. In the present invention, the extracellular matrix layer may contain an effective amount of an extracellular matrix or a component or partial peptide thereof and a non-cell adhesive support (for example, hydrogel). The extracellular matrix may be a laminin-derived peptide or a laminin α-1 chain-derived peptide, for example, a peptide consisting of the amino acid sequence of 2116 to 2120 positions of the sequence registered under GenBank registration number KAI4045526.1 or a part of laminin containing the amino acid sequence, or a peptide consisting of the amino acid sequence of 950 to 954 positions of the sequence registered under GenBank registration number EAL24388.1 or a part of laminin containing the amino acid sequence. The extracellular matrix layer may contain vitronectin (for example, human vitronectin having, for example, the amino acid sequence under GenBank registration number EAW51082.1) or a part (VTN-N) thereof.

The thickness of the extracellular matrix layer from the inner bottom surface to the top surface of the extracellular matrix layer in contact with an embryo can be appropriately set by those skilled in the art based on the above disclosure. For example, the thickness can be 50 µm or more, 100 µm or more, 200 µm or more, 300 µm or more, 400 µm or more, 500 µm or more, 600 µm or more, 700 µm or more, 800 µm or more, 900 µm or more, or 1000 µm or more. If the thickness is set in this way, it is possible to prevent the cells contained in a blastula to be directly in contact with the bottom surface.

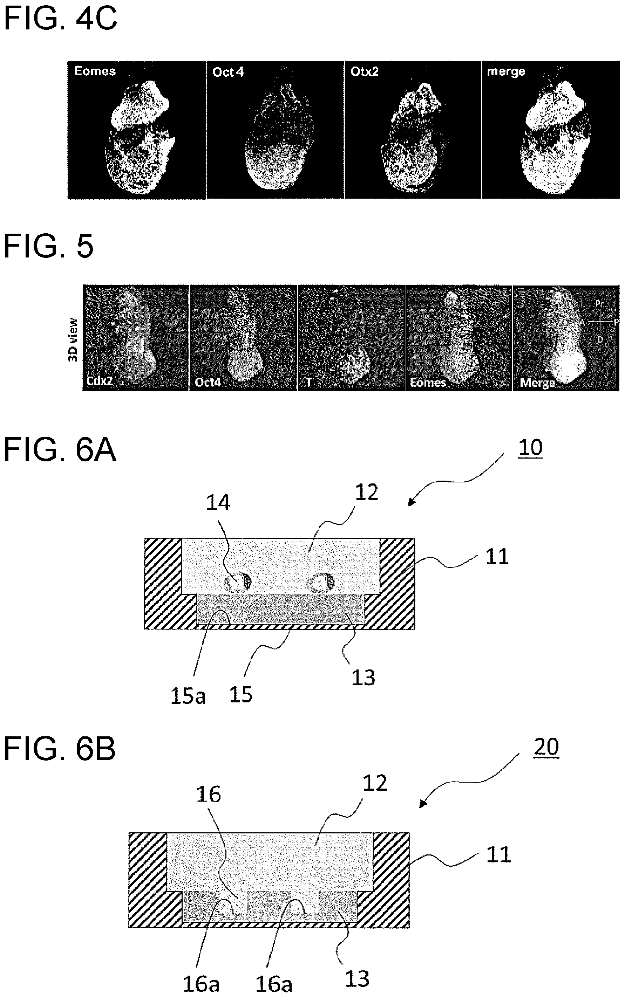

A culture dish 10 having an inner bottom surface coated with an extracellular matrix layer and to be used in the method of the present invention will be explained with reference to Figure 6A. Figure 6A shows a sectional view of a culture dish when an embryo is cultured. The culture dish 10 has a bottom 15 and a side wall 11 extending upward from the outer periphery of the bottom 15. The culture dish 10 has an extracellular matrix layer 13 on the inner bottom surface 15a. More specifically, the culture dish 10 has the inner bottom surface 15a coated with an extracellular matrix layer 13. The extracellular matrix layer has a sufficient thickness as mentioned above. An embryo 14 is placed on the extracellular matrix layer and cultured in this state. In the method of the present invention, it is considered unnecessary that contact of the embryo 14 to be cultured to the inner bottom surface 15a is completely blocked by the extracellular matrix layer but contact of the embryo 14 to be cultured to the inner bottom surface 15a can be completely blocked by the extracellular matrix layer by the aforementioned constitution.

In the method of the present invention, the shape of the extracellular matrix layer is not limited as long as it can completely block the contact of the embryo 14 to be cultured to the inner bottom surface 15a. For example, in the method of the present invention, a culture dish 20 shown in Figure 6B can be used. For example, as shown in Figure 6B, the extracellular matrix layer 13 may have a sufficient large well 16 for introducing the embryo 14, in the top surface. The hole 16 may be, for example, approximately cylindrical hole. In this case, the thickness of the extracellular matrix layer from the bottom 15a to top surface 16a of the extracellular matrix in contact with an embryo can be set as described above. In the method of the present invention, it is considered unnecessary that contact of the embryo 14 to be cultured to the inner bottom surface 15a is completely blocked by the extracellular matrix layer but contact of the embryo 14 to be cultured to the inner bottom surface 15a can be completely blocked by the extracellular matrix layer. The depth of the hole can be larger than the length of the embryo. The hole may have an opening sufficiently large to put the embryo. The hole may have a shape sufficiently large to put the embryo. The embryo may be in contact with the bottom and/or side surface of the hole during culture.

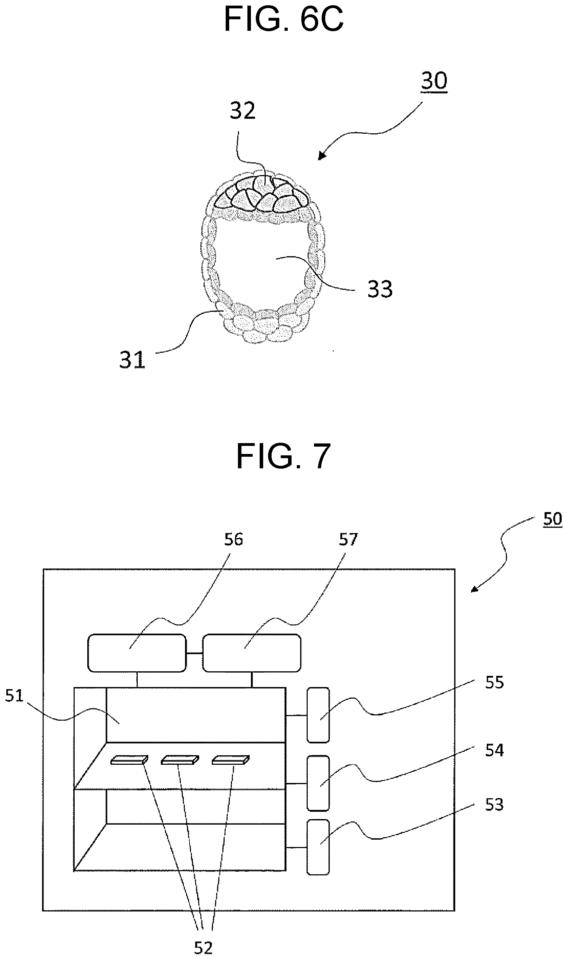

In the method of the present invention, the embryo may be placed on the extracellular matrix layer in any orientation as long as it can be normally developed. In an embodiment of the present invention, an embryo can be placed on the extracellular matrix layer such that the mural trophectoderm faces downward or such that mural trophectoderm membrane faces upward and inner cell mass faces downward. For example, in an embodiment, the embryo may be a rodent (for example, a mouse or a rat) embryo and can be placed on the extracellular matrix layer such that the mural trophectoderm faces downward (for example, in Figure 6C, the inner cell mass faces upward and the mural trophectoderm membrane faces downward). In a preferred embodiment of the present invention, the embryo is a primate (for example, human) embryo and placed on the extracellular matrix layer such that the mural trophectoderm membrane faces upward and the inner cell mass faces downward. As shown in Figure 6C, a blastocyst 30 is constituted of an inner cell mass 32, a blastocoel 33 and trophectoderm 31 that envelops the inner cell mass 32 and blastocoel 33. The mural trophectoderm is not located near the inner cell mass 32 side but located near the blastocoel 33 side. In other words, the blastocyst can be arranged such that the inner cell mass 32 faces upward and a blastocoel 33 faces downward.

In a preferred embodiment of the present invention, an embryo can be cultured such that the embryo is not in contact with a solid support (particularly an artificial solid support, preferably a cell adhesive solid support) except for the extracellular matrix layer. In an embodiment of the present invention, an embryo is cultured in the absence of a feeder.

In the method of the present invention, the condition suitable for culture can be appropriately determined by those skilled in the art. For example, the condition suitable for culture can be a condition of 35°C to 39°C (for example, about 37°C) and about 3 to 7% CO₂ (for example, about 5% CO₂). In an embodiment, for example, the condition suitable for culture can be a condition of 35°C to 39°C (for example, about 37°C), about 3 to 7% CO₂ (for example, about 5% CO₂) and about 15 to 25% O₂ (for example, about 20% O₂). In an embodiment, the condition suitable for culture can be a condition of about 37°C, about 5% CO₂ and hypoxia. The hypoxia refers to a condition of a significantly lower oxygen partial pressure than an oxygen partial pressure (about 20%) in the atmosphere. The hypoxic condition can be, for example, a condition of 5% to 150 oxygen partial pressure.

Examples of the medium to be used in the method of the present invention include a serum-containing medium, a serum-free medium and a chemically defined medium. As the medium, any medium can be used as long as it is known as a medium suitably used for culturing an embryo. The medium may contain additives including a serum substitute such as KnockOut^{™} serum substitute, L-glutamine (for example, L-alanine-L-glutamine dipeptide), an antibiotic substance (for example, penicillin, streptomycin), insulin, transferrin, selenium, ethanolamine, reducing agent (for example, monothioglycerol), and L-cysteine (for example, N-acetyl-L-cysteine) , in addition to a basal medium (for example, a basal medium used for culturing an embryo such as DMEM, DMEM/F12, and Advanced DMEM/F12). In a preferred embodiment, the medium to be used in the method of the present invention can be a serum-free medium including Advanced DMEM/F12. In an embodiment, the medium contains serum. In an embodiment, the medium contains human serum or at least one of the components thereof. In an embodiment, the medium may be a mixture containing human serum or at least one of the components thereof and a chemically defined medium. In an embodiment, the medium is a xeno-free medium containing serum. In an embodiment, the medium is a xeno-free medium containing human serum or at least one of the components thereof. In an embodiment, the xeno-free medium can be a mixture consisting of human serum or at least one of the components thereof and a chemically defined medium.

The present invention can be used for examining the effect of a test compound on implantation and/or development of an embryo. For example, an embryo such as a normal embryo and an abnormal embryo is cultured in the presence of a test compound. In the case where the development of the embryo differs from that of an embryo cultured in the absence of a test compound, the test compound is determined to have a physiological activity on the embryonic development. In this manner, the effects of a test compound on the development of a normal embryo and, the effects of a test compound on an abnormal embryo can be examined. A compound that normalizes abnormal development of an embryo with implantation failure or an embryo abnormally implanted can be used as a drug for infertility treatment. Accordingly, the method of the present invention can be used for screening a drug for infertility treatment. The test compound herein refers to a compound to be tested, more specifically, a compound whose effect on implantation and/or development of an embryo is to be confirmed. The test compound may be a compound having an effect on implantation and/or development of an embryo, a compound suspected to have an effect on implantation and/or development of an embryo, or a compound having no effect on implantation and/or development of an embryo. This is because even if a compound does not have an effect on its own it may have an effect depending on the form in which it exists, for example, depending on the content of the composition containing the compound.

The present invention can be used for examining whether a test embryo has an ability to develop. For example, according to the method of the present invention, a test embryo in the first development phase is cultured under the condition where a normal embryo in the first development phase develops from the first development phase to the second development phase. If the test embryo is not normally developed from the first development phase to the second development phase, the test embryo is determined to have an abnormality in development of the embryo in the first development phase to the second development phase; and/or, if the test embryo is normally developed from the first development phase to the second development phase, the test embryo is determined to have an ability to develop from the first development phase to the second development phase. As mentioned above, whether an embryo has a developmental abnormality or not can be examined. In this way, it is possible to examine whether a subject is qualified to receive, for example, an infertility treatment. The test embryo herein refers to an embryo to be tested, for example, an embryo to be checked for implantation and/or developmental ability.

According to the present invention, it is possible to further develop an embryo from a fertilized egg to a later development stage than a gastrula by using another method for developing a fertilized egg to a blastocyst and a method for developing an embryo from a gastrula to a further later development stage, in combination. The method for developing a fertilized egg to a blastocyst was developed in mice (Manipulating the Mouse Embryo: a Laboratory Manual 4th ed. (Cold Spring Harbor Laboratory Press, 2014) and has been clinically applied to human *in-vitro* fertilization (Steptoe et al., Lancet, 1978). For example, those skilled in the art can prepare a blastocyst from a fertilized egg by differentiating a fertilized egg to a blastocyst under a condition suitable for differentiation based on the above description. A method for developing a gastrula to a later development phase (hindlimb formation stage: E11), includes, for example, a rotational culture method using human serum and rat serum in a medium (Aguilera-Castrejon et al., Nature, 2021). For example, those skilled in the art can prepare an embryo of the third development phase from a gastrula by differentiating a gastrula under the condition suitable for differentiation of a gastrula into the hindlimb formation stage, based on the above. According to the present invention, it is expected that a method for reproducing the whole step from a fertilized egg to an embryo in the hindlimb formation stage, *in vitro,* can be established without destroying an embryo, in this way. Accordingly, the present invention provides an *in-vitro* method for obtaining an embryo in the hindlimb formation stage from a fertilized egg.

The present invention provides a culture system (culture device) 50 for use in culturing an embryo, comprising
a culture dish 52, which has an inner bottom surface coated with an extracellular matrix layer,
wherein a thickness of the extracellular matrix layer from the bottom to a top surface of the extracellular matrix layer in contact with an embryo is a predetermined thickness or more; the predetermined thickness is sufficient to prevent adhesion of the embryo onto the bottom surface during culture or adhesion of the embryo and expansion of an adhesion area thereof on the bottom surface,
the embryo is an embryo at a point in a first development phase between the blastocyst stage and the gastrula stage, and
whereby, an embryo in the blastocyst stage can be developed into a second development phase between the blastocyst stage and the gastrula stage, and the second development phase is the same as the first development phase or a later development phase.

The culture system can be used for developing the embryo from a first development phase to a second development phase. The details of the first development phase and second development phase are as mentioned above.

The structure of the culture system will be described with reference to Figure 7, as an example. As shown in Figure 7, the culture system has a storage cabinet 51 storing culture dishes 52 and further a thermometer 53 measuring the temperature in the storage cabinet 51, a hygrometer 54 measuring the humidity in the storage cabinet, and a thermostat 55 controlling the temperature in the storage cabinet. In a preferred embodiment, the interior of the culture dish 52 is controlled to have a sufficient humidity. The humidity can be controlled by arranging, for example, a means for maintaining a moisture content (for example, sponge or tissue keeping a moisture content) in the space where the culture dishes 52 are present. In an embodiment, the culture dishes 52 is a multiwell plate, which is further arranged in an open container (for example, an open container with an openable lid, for example, a petri dish)(see, Figure 1A and 1C). Furthermore, the means for maintaining a moisture content is arranged within the open container and can control the humidity of the open container (for example, petri dish) covered with a lid. In a preferred embodiment, the culture system 50 has a gas concentration sensor 56 for the interior of the storage cabinet 51. The gas concentration sensor 56 can measure carbon dioxide and/or oxygen concentrations. In a preferred embodiment, the culture system may be further equipped with an oxygen concentration controller 57 controlling the concentration of oxygen based on the measured oxygen concentration. The oxygen concentration controller 57 can supply nitrogen to produce a hypoxia condition within the storage cabinet.

According to the present invention, there are provided embryos in the late blastocyst to egg-cylinder embryo stages, and embryos in the egg-cylinder embryo to gastrula stages obtained by the method of the present invention, and cultures containing the embryos obtained by the method of the present invention.

### Examples

### Materials and Methods

### Mouse embryo

Female mice of 3 ± 1 months old were subjected to natural mating. The noon of the day on which vaginal plug was detected was specified as E0.5. This study has gained the approval by the Animal Experiment Committee of Kyushu University.

Mouse embryos were collected by the M2-medium (Sigma-Aldrich M7167)flushing the uteri of ICR female mice mated with ICR males, B6C3 female mice mated with hybrid mice (B6C3) of C57BL/6N and C3H/HeN, and B6C3 female mice mated with B6C3-Otx2^{em1 (tdTomato) Utr} males at embryonic day E3.5 or E4.5 (12: 00) by sending M2 medium (Sigma-Aldrich M7167), and cultured using a culture medium (*in-vitro* culture medium (IVCM): modified IVC2 medium (Bedzhov et al.2014)). The composition of IVCM was as follows: advanced DMEM/F12 (Thermo Fisher Scientific, 12634010), 30% KSR (Thermo Fisher Scientific, 10828010), 1× GlutaMAX I (Thermo Fisher Scientific, 35050061), 1× penicillin-streptomycin (Thermo Fisher Scientific, 15140122), 1× ITS-X (Thermo Fisher Scientific, 51500056), 1× monothioglycerol (FUJIFILM, 195-15791), 200 ng/ml progesterone (Sigma-Aldrich, P8783), and 25 µM N-acetyl-L-cysteine (Sigma-Aldrich, A7250). This step was carried out by a stereo microscope (Leica Co., Ltd.) equipped with ThermoPlate (TPiD-TLDX manufactured by Tokai Hit., Co, Ltd) at 37°C.

### Culture of Mouse Embryo

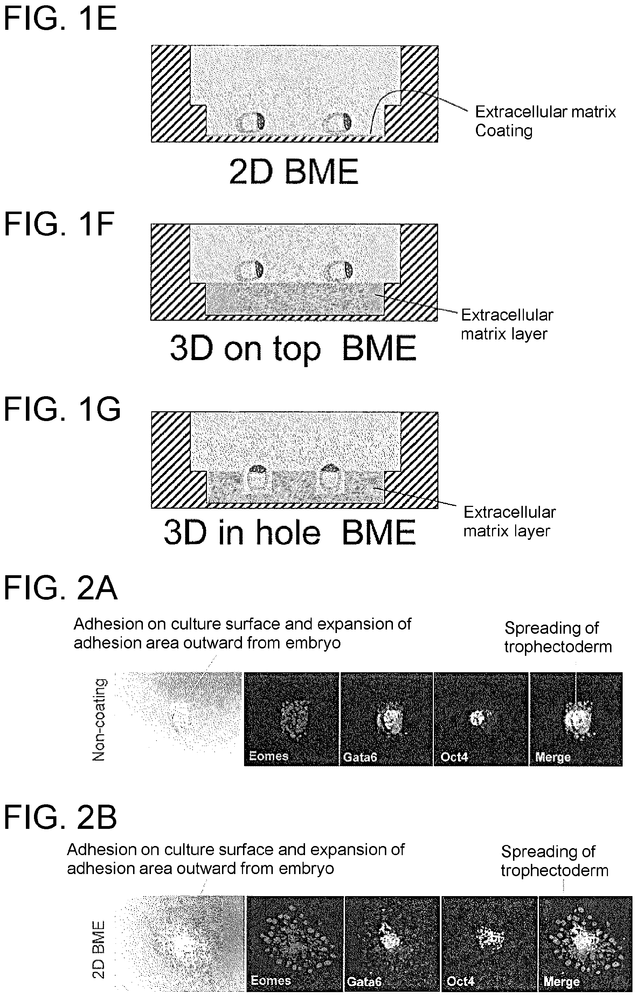

Embryos were cultured using µ-Slide 8 Well (Ibidi, 80826) or µ-Slide Angiogenesis (Ibidi, 81506), which was placed together with wet tissue in a 10-cm petri dish (Figure 1A-C). When an embryo (E3.5) was cultured, as previously mentioned, zona pellucida was removed by acidic Tyrode's solution (Sigma-Aldrich, T1788) (Bedzhov et al.) as previously mentioned. The zona-pellucida free blastocyst was cultured in µ-Slide 8 Well using IVCM supplemented with 8 nM β-estradiol (Sigma-Aldrich, E8875) and 10 ng/ml osteopontin (Biolegend, 763602) at 37°C, in an environment of 5% CO₂ for 24 hours. An embryo (E4.5) or an embryo (E3.5) previously cultured was cultured with IVCM in µ-Slide Angiogenesis at 37°C and 5% CO₂. In the 2D culture, the embryo was cultured on the µ-Slide Angiogenesis coated (Figure 1E) or not coated (Figure 1D) with an extracellular matrix, i.e., a basement membrane extract (BME) (R&D systems, Cultrex^{™} UltiMatrix Reduced Growth Factor, BME001). Coating with BME was performed on a cooling module (Corning CoolBox XT, 432022). After BME was placed on µ-Slide Angiogenesis and immediately removed, the µ-Slide was placed in an incubator of 37°C for 10 minutes to form a gel (Figure 1E). In the 3D on top culture or 3D in hole culture, 10 µl (for 3D on top experiment) or 15 µl (for 3D in hole experiment) of BME was put in inner wells of the µ-Slide Angiogenesis and incubation was performed at 37°C for 10 minutes (Figure 1F, 1G). In the 3D in hole culture, holes of 150 to 200 µm in diameter were formed in the gel by use of a micropipette tip (Figure 1G). Embryos were cultured in holes with the mural trophectoderm face downward. Thirty hours after the initial culture, the medium was exchanged with IVCM previously warmed and culture was continued at 37°C and 5% CO₂. Thereafter, the medium was exchanged at 12: 00 every day. Images were taken by a stereoscopic microscope (Leica) or BZ-X700 microscope (KEYENCE CORPORATION). In all cases of culturing embryos, the forementioned chemically defined medium was used. To the chemically defined medium, serum such as fetal bovine serum was not added.

### Immunostaining

Embryos were fixed with 4% paraformaldehyde at room temperature for 20 minutes. The embryos fixed were made transparent in accordance with the CUBIC protocol (Tainaka et al., 2018) modified as shown below. The fixed embryos were treated with CUBIC-L (T3740, Tokyo Kasei Kogyo Co., Ltd.) in an incubator (hybridization incubator) equipped with a shaker at 37°C, overnight. To terminate the treatment for transparency, a shaking operation using PBS at room temperature was gently performed twice and washing was individually performed for 30 minutes. Incubation with a primary antibody was performed with a seesaw shaker at 4°C overnight. Thereafter, incubation was made using fluorescently labeled AlexaFluor secondary antibody (Thermo Fisher Scientific) at room temperature for 2 hours. The primary antibody and secondary antibody both were diluted with 1% BSA and 0.1% Tween-PBS. Soaking was made with CUBIC-R+ (T3741, Tokyo Kasei Kogyo Co., Ltd.) and incubation was made by a hybridization incubator at 37°C, overnight. Images were obtained by an inverted SP8 confocal microscope (Leica) and analyzed by LAS X software (Leica) or Imaris software (Oxford instruments).

### Results

For embryogenesis, a method for culturing a preimplantation embryo (e.g., Bedzhov et al., Nature Protocols 9, 2732-2739, 2014) and a method for culturing a post-implantation embryo (e.g., Aguilera-Castrejon et al., Nature 593, 119-124, 2021) have been developed. In the case of mouse, the preimplantation embryo is an embryo up to E4.5 from fertilization, whereas the post-implantation embryo is an embryo of E5.0 or later. Bedzhov et al. (2014) disclose a method for developing a blastocyst of E3.5 to E4.5 *in vitro* beyond the implantation stage, while Aguilera-Castrejon et al. (2021) disclose a method for reproducing normal development from E5.5 to E11 *in vitro.* However, it has not succeeded in developing a preimplantation embryo efficiently up to the gastrula stage *in vitro.* An embryo (E4.5) corresponds to a blastocyst-stage embryo and E5.5 embryo corresponds to an egg-cylinder embryo (Egg cylinder).

As shown in Figure 1A, wet tissue was placed along the side wall of a 10-cm petri dish and µ-slide 8 well was placed at the center of the petri dish. As shown in Figure 1B, the µ-slide angionegesis has wells and embryos can be placed in individual wells. As shown in Figure 1C, wet tissue was placed along the side wall of a 10-cm petri dish and an µ-slide angionegesis was placed at the center of the petri dish.

The individual wells were coated differently. A well having a non-coating bottom (non-coating), as shown in Figure 1D; a well having a bottom surface having a membrane of an extracellular matrix, i.e., a basement membrane extract (BME) formed thereon (2D BME), as shown in Figure 1E; a well into which a block having a thick basement membrane matrix was introduced (3D on top), as shown in Figure 1F; and a well into which a block having a thick basement membrane matrix with holes each small enough to accommodate an embryo was introduced (3D in hole), as shown in Figure 1G, were formed, and then, an IVCM medium was introduced. Mouse embryos in the blastocyst stage (an embryo (E4.5)) were introduced in individual wells. The embryos were derived from ICR female mice. In the 3D in hole culture, the embryos were embedded in the small holes. Thereafter, culture was performed at 37°C for a day. The embryos cultured for a day were observed by an optical microscope and a fluorescence microscope.

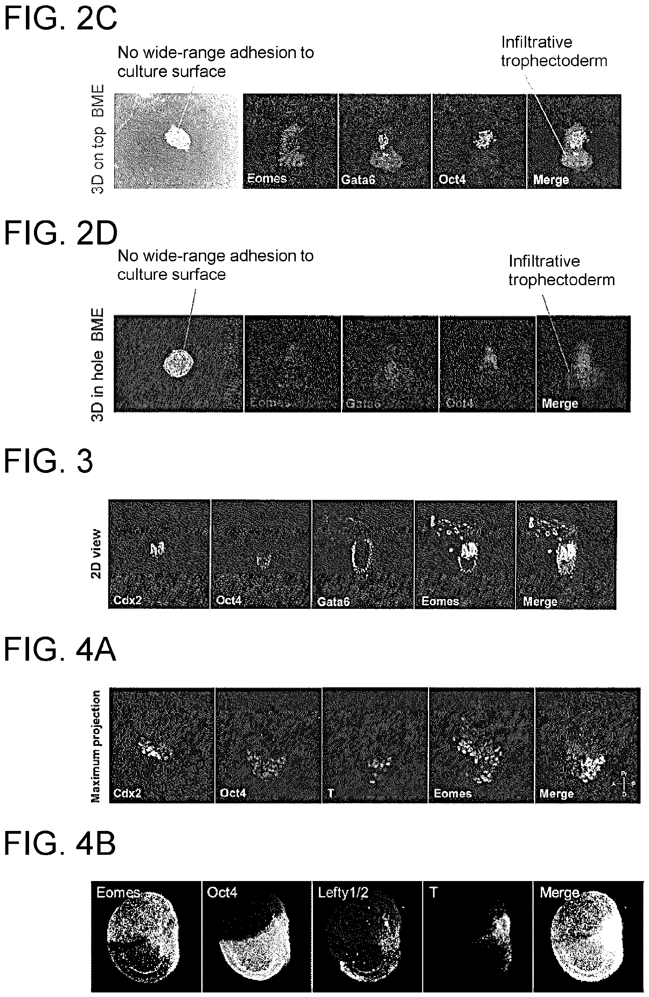

The results were shown in Figures 2A to D. As shown in Figure 2A and 2B, when the embryos were cultured in wells having a non-coating bottom, they adhered onto the plate surface and changed into flat form. It was found that the trophectoderm expanded on the plate. In contrast, the embryos subjected to 3D on top culture and 3D in hole culture were normally developed. In the case of the embryos subjected to 3D on top culture and 3D in hole culture, adhesion and expansion of the embryos on the plate surface were not observed. Table 1 shows the ratio of embryos normally developed (embryos corresponding to E5.0 to E5.3).

**[Table 1]**

| Table 1 The ratio of embryos (E4.5) normally developed after in-vitro culture for a day | | | | |
|---|---|---|---|---|
| | No coating (Figure 2A) | 2D BME (Figure 2B) | 3D on top (Figure 2C) | 3D in hole (Figure 2D) |
| Ratio of embryos normally developed | 0/4 | 0/4 | 3/7 | 4/4 |

As shown in Table 1, it was revealed that the ratio of the embryos normally developed in the 3D in hole culture is prominently large compared to embryos normally developed in the cultures under the other coating conditions.

Late blastocysts (E4.5) were cultured in an IVCM medium by the 3D in hole culture for 2 days. When observed by an optical microscope, the cultured embryos had cylindrical (egg cylinder) shapes. The cultured embryos were subjected to immunohistochemical staining. In the immunohistochemical staining, expressions of Cdx2, Oct4, Gata6 and Eomes were detected by using primary antibodies and labeled secondary antibodies specifically binding to them, respectively. The results were as shown in Figure 3. As shown in Figure 3, each of the embryos cultured by the 3D in hole culture for 2 days was formed into a Gata6-positive parietal endoderm. The parietal endoderm forms Reichert's membrane. In contrast, in the previous report (Bedzhov et al., Nature Protocols 9, 2732-2739, 2014), the formation of Reichert's membrane was not confirmed.

Late blastocysts (E4.5) were cultured in the IVCM medium by the 3D in hole culture for 3 days. The embryos were derived from ICR female mice. The cultured embryos were subjected to immunohistochemical staining. In the immunohistochemical staining, expressions of Cdx2, Oct4, T and Eomes were detected by using primary antibodies and labeled secondary antibodies specifically binding to them, respectively. "T" is a marker for the primitive streak and mesoderm; and Eomes is a marker for the primitive streak and the extraembryonic ectoderm. The obtained embryos were developed up to the early primitive streak stage. The results of the immunohistochemical staining were as shown in Figure 4A. As shown in Figure 4A, expression of T and Eomes were observed in the embryos cultured for 3 days. From this, it was found that the primitive streak was formed in the embryos. Furthermore, it was revealed that the embryos were developed up to the gastrula stage. In the previous report (Bedzhov et al., Nature Protocols 9, 2732-2739, 2014), the formation of primitive streak was not disclosed. The development efficiency of embryos in the development phase equivalent to E6.5 in the above condition was 1/5. Then, culture was performed in hIVCM medium prepared by adding human serum in place of KSR of the IVCM medium, in the same condition as above. The composition of the hIVCM medium was as follows: advanced DMEM/F12 medium (Thermo Fisher Scientific) containing 30% human serum (Kohjin Bio), 1× GlutaMax I (Thermo Fisher Scientific), 1× penicillin-streptomycin (Thermo Fisher Scientific), 1× ITS-X (Thermo Fisher Scientific), 200 ng/mL progesterone (Sigma-Aldrich, P8783), and 10 µM Mito-TEMPO (Selleck, S9733). As a result, the development efficiency of embryos at E4.5 to the development phase equivalent to E6.5 (see, Figure 4B) increased up to 3/5. The 3D in hole culture was further continued for 4 days. As a result, as shown in Figure 4C, embryos in the development phase equivalent to E7.5 (late gastrulation stage embryo) were obtained. As the characteristics of the embryos obtained in Figure 4C, the expression of Otx2, an ectodermal marker, on the ventral side, as indicated by the area outlined in white dashed lines was detected, which suggests embryonic development up to the late gastrulation stage.

Blastocysts (E3.5) were cultured for 4 days. The embryos were derived from ICR female mice. More specifically, the embryos were cultured in the IVCM medium containing β-estradiol and osteopontin for the first day and then in the 3D on top for the following 3 days. The embryos thus obtained were grown to the early primitive streak stage. The results of the immunohistochemical staining were as shown in Figure 5. As shown in Figure 5, T and Eomes were expressed in the embryos cultured for 3 days. From this, it was found that the embryos formed the primitive streak. Furthermore, it was revealed that the embryos were developed up to the gastrula stage.

According to the Example, the embryonic development phase from E4.5 (late blastocyst) to E7.5 (late gastrulation embryo) including before and after implantation was successfully reproduced *in vitro.*

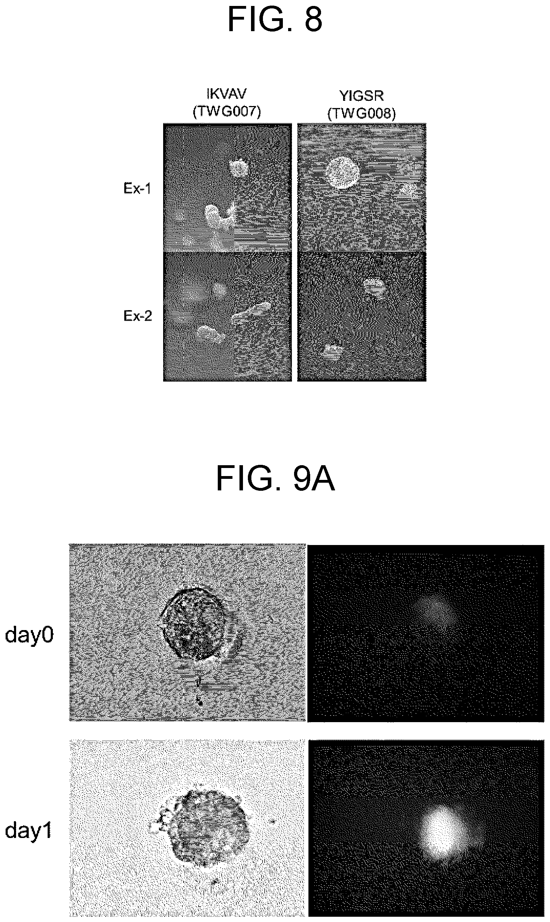

To analyze the effect of the basement membrane component on embryonic development, 3D in hole cultures of the same embryos as mentioned above were performed using VitroGel (TWG007: IKVAV, TWG008: YIGSR, TheWell Bioscience Inc.) in place of the basement membrane component. VitroGel and a culture solution (3: 1) were mixed, and the obtained mixed solution was transferred to wells of the inner wells of µ-Slide Angiogenesis and then gelatinized. After incubated at room temperature for 20 minutes, the medium was added onto the gel. In the 3D in hole culture, holes of 150 to 200 µm in diameter were formed in the gel by use of a micropipette tip. Embryos (E4.5) were cultured in IVCM for 72 hours. The embryos were derived from B6C3 female mice mated with a hybrid male mouse (B6C3) of C57BL/6N and C3H/HeN. The results were as shown in Figure 8. As shown in Figure 8, a gel containing the above peptide, which is a partial peptide derived from laminin, supported embryonic development. From this, it was revealed that the hydrogel containing the laminin-derived peptide can play a role as the extracellular matrix layer (or support).

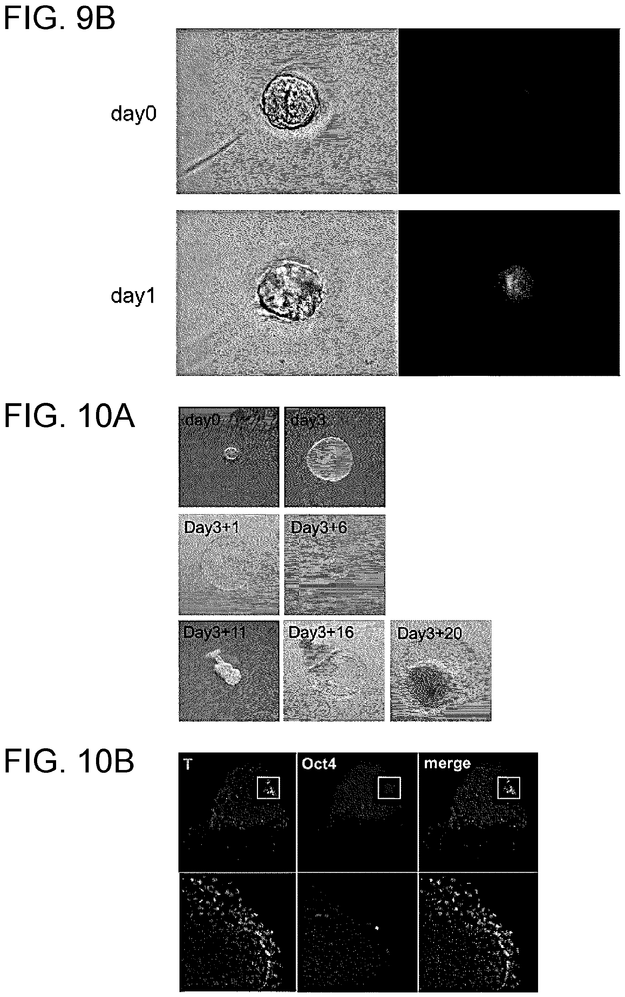

For the cultures by the 3D on Top and 3D in Hole, Gelrite (gellan gum; FUJIFILM Wako Pure Chemical Corporation) was used. Gelrite is a non-cell adhesive support. Gelrite powder was dissolved in water (0.8%) and thereafter, boiled in a microwave. For forming a Gelrite gel, a mixture of 0.8% Gelrite in 12 µl of water was transferred to the wells of the inner wells of µ-Slide Angiogenesis. As the extracellular matrix, BME001 or recombinant human vitronectin (VTN-N) was used. The recombinant human vitronectin (VTN-N) is derived from the region of 62 to 478 positions of human vitronectin. The solution was incubated at room temperature for 20 minutes, and thereafter, 1% BME001/culture medium or 5 µg/mL VTN-N was added onto the gel as the extracellular matrix to deeply impregnate Gelrite with the extracellular matrix sufficiently by performing incubation overnight. For 3D in hole culture, holes of 70 to 100 µm in diameter were formed in the gel impregnated with the extracellular matrix by use of a micropipette tip. The embryos were in contact with Gelrite containing the extracellular matrix in this condition but not in contact with the bottom of the wells. Embryos (E4.5) (tdTomato was operably connected downstream of the Otx2 gene) were cultured in hIVCM by 3D in hole for 24 hours. The results were as shown in Figures 9A and B. The embryos were derived from B6C3 females mated with B6C3-Otx2^{em1 (tdTomato) Utr} males. As shown in Figures 9A and B, both cases where BME001 (Figure 9A) and VTN-N (Figure 9B) were used as the extracellular matrix, the embryos normally developed.

Otx2 is a marker for a post-implantation embryo, expressed in the epiblast, and expression of tdTomato gene introduced downstream of the Otx2 gene suggests that the embryos developed into the post-implantation development phase. As shown in Figures 9A and B, one day after initiation of the 3D in hole culture, tdTomato was expressed by being driven by Otx2 promoter in all embryos, which suggested that embryos were developed up to post-implantation embryos. From the above, it was suggested that the basement membrane extract supports embryonic development. It was revealed that a hydrogel containing BME001 or VTN-N (basement membrane component) plays a role as the extracellular matrix layer (or support).

The above experiments were performed using mouse embryos. In the following, primate embryos were cultured by the method of the present invention.

Embryos (blastocysts) of common marmosets (Callithrix jacchus) were provided by favor of Dr. Erika Sasaki (Central Institute for Experimental Animals). To thaw frozen embryos in a vial, 900 µL of SPB2 (pre-warmed at 38°C, under the condition of 5% CO₂ and 5% O₂ for one hour or more) was added to the vial and the embryos were washed three times with PB1. The embryos were transferred to droplets of ORIGIO Sequential Blast containing 20% FBS and 2 mM L-glutamine medium (preheated at 38°C, under the condition of 5% CO₂ and 5% O₂ for one hour or more) and cultured at 38°C, under the condition of 5% CO₂ and 5% O₂ until the embryos were hatched. Thereafter, the embryos were cultured in the hIVCM medium by the 3D on Top method. A half of the medium was exchanged with the hIVCM medium containing 10 nM 4-hydroxyestradiol, and culture was performed for 5 days at 38°C under the condition of 5% CO₂ and 5% O₂. Thereafter, a half of the medium was exchanged with the hIVCM medium and culture was performed at 37°C and 5% CO₂. Half of the medium was exchanged at 12:00 every day. The images of the obtained embryos were taken by a stereoscopic microscope (Leica). As a result, the embryos were successfully cultured over 20 days as shown in Figure 10A. When the embryos were immunostained with respect to a primitive streak marker, T, it was found that the development phases of embryos reached the gastrulation stage, as shown in Figure 10B. From this, it was revealed that the embryo of the primate can be developed *in vitro* from the pre-implantation to the post-implantation embryo.

Bedzhov, I., Leung, C.Y., Bialecka, M., and Zernicka-Goetz, M. (2014). In vitro culture of mouse blastocysts beyond the implantation stages. Nat Protoc 9, 2732-2739. Tainaka, K., Murakami, T.C., Susaki, E.A., Shimizu, C., Saito, R., Takahashi, K., Hayashi-Takagi, A., Sekiya, H., Arima, Y., Nojima, S., et al. (2018). Chemical Landscape for Tissue Clearing Based on Hydrophilic Reagents. Cell Reports 24, 2196-2210.e2199.

## Claims

1. A method for culturing an embryo *in vitro,* comprising
providing a culture dish having an inner bottom surface coated with an extracellular matrix layer on which the embryo is placed, wherein the embryo is contained in a sufficient amount of a medium suitable for culturing the embryo, and then
culturing the embryo placed on the extracellular matrix layer under a condition suitable for culturing the embryo, wherein
a thickness of the extracellular matrix layer from the inner bottom surface to a top surface of the extracellular matrix layer in contact with the embryo is equal to or more than a predetermined thickness;
the predetermined thickness is sufficient to prevent adhesion of the embryo onto the inner bottom surface during culture or adhesion of the embryo and expansion of an adhesion area thereof on the inner bottom surface;
the embryo is an embryo in a first development phase between a blastocyst stage and a gastrula stage,
thereby successfully developing the embryo in the blastocyst stage up to a second development phase between the blastocyst stage and the gastrula stage; and the second development phase is the same development phase as the first development phase or a later development phase.

2. The method according to claim 1, wherein the medium is a serum-free medium.

3. The method according to claim 1 or 2, wherein the medium is a chemically defined medium.

4. The method according to any one of claims 1 to 3, wherein a site on which the embryo is placed is within a hole formed in a top surface of the extracellular matrix layer.

5. The method according to claim 4, wherein the embryo is placed so as to be in contact with an inner wall of the hole.

6. The method according to any one of claims 1 to 5, wherein the embryo is placed on the extracellular matrix layer such that a mural trophectoderm thereof faces up or down.

7. The method according to any one of claims 1 to 6, wherein the first development phase is a late blastocyst and the second development phase is a gastrula.

8. The method according to any one of claims 1 to 7, wherein
the medium contains a test compound,
the method further comprises
confirming whether or not growth of the embryo cultured in the presence of the test compound differs from growth of the embryo cultured in the absence of the test compound,
if there is a difference, the test compound is determined to have an effect on development of the embryo in the first development phase to the second development phase, and/or
if there is no difference, the test compound is determined to have no effect on development of the embryo in the first development phase to the second development phase.

9. The method according to any one of claims 1 to 7, further comprising
culturing a test embryo in the first development phase under a condition where a normal embryo in the first development phase develops from the first development phase to the second development phase, wherein
if the test embryo is not normally developed from the first development phase to the second development phase, the test embryo is determined to have an abnormality in development of the embryo in the first development phase to the second development phase, and/or
if the test embryo is normally developed from the first development phase to the second development phase, the test embryo is determined to have an ability to develop from the first development phase to the second development phase.

10. A culture system for use in culturing an embryo, comprising
a culture dish for culturing the embryo, the culture dish having an inner bottom surface coated with an extracellular matrix layer, wherein
a thickness of the extracellular matrix layer from the bottom surface to a top surface of the extracellular matrix layer in contact with the embryo is equal to or more than a predetermined thickness;
the predetermined thickness is sufficient to prevent adhesion of the embryo onto the bottom surface during culture or adhesion of the embryo and expansion of an adhesion area thereof on the bottom surface;
the embryo is an embryo in a first development phase between a blastocyst stage and a gastrula stage,
thereby successfully developing the embryo in the blastocyst stage up to in a second development phase between the blastocyst stage and the gastrula stage; and the second development phase is the same development phase as the first development phase or a later development phase.
